# EUROPÄISCHE PATENTSCHRIFT

(11) **EP 3 831 816 B1**
(45) Veröffentlichungstag und Bekanntmachung des Hinweises auf die Patenterteilung: **02.08.2023**
(21) Anmeldenummer: 20210923.7
(22) Anmeldetag: 01.12.2020
(51) Int. Cl.: C07D 271/12

(54) **VERFAHREN ZUR SYNTHESE VON 4,6-DINITROBENZOFUROXAN**
METHOD FOR THE SYNTHESIS OF 4,6-DINITROBENZOFUROXANE
PROCÉDÉ DE SYNTHÈSE DU 4,6-DINITROBENZOFUROXANE

(30) Priorität: 03.12.2019 DE 102019132863
(43) Veröffentlichungstag der Anmeldung: 09.06.2021
(73) Patentinhaber: Dynitec GmbH, 53840 Troisdorf (DE)
(72) Erfinder: SCHULZ, Björn, 53844 Troisdorf, DE (DE); SCHIRRA, Rainer, 53797 Lohmar, DE (DE); BARGON, Stephan, 53842 Troisdorf, DE (DE); VIGNOLD, Burkhardt, 53844 Troisdorf, DE (DE)
(74) Vertreter: Fitzner, Uwe

(56) Entgegenhaltungen:
- Dong Yan: "Preparation of 5,7-diamino-4,6-dinitrobenzenfuroxan", Baopo Qicai, 1. Januar 2013 (2013-01-01), Seiten 10-13, XP055785914, DOI: 10.3969/j.issn.1001-8352.2013.01.003 Gefunden im Internet: URL:http://d.wanfangdata.com.cn/periodical /bpqc201301003 [gefunden am 2021-03-15]
- Drost: "Über Nitroderivate des o-Dinitrosobenzols", Justus Liebig's Annalen der Chemie, 1. Januar 1899 (1899-01-01), Seiten 49-69, XP055785866, Gefunden im Internet: URL:https://chemistry-europe.onlinelibrary .wiley.com/doi/abs/10.1002/jlac.1899307010 4 [gefunden am 2021-03-15]
- MACCORMACK P ET AL: "UNUSUAL STRUCTURE IN MEISENHEIMER COMPLEX FORMATION FROM THE HIGHLY ELECTROPHILIC 4,6-DINITROBENZOFUROXAN", JOURNAL OF ORGANIC CHEMISTRY, AMERICAN CHEMICAL SOCIETY, WASHINGTON, Bd. 53, Nr. 18, 1. Januar 1988 (1988-01-01), Seiten 4407-4409, XP001057761, ISSN: 0022-3263, DOI: 10.1021/JO00253A044
- SHINDE P.D. ET AL: "Some Transition Metal Salts of 4,6-Dinitrobenzofuroxan: Synthesis, Characterization and Evaluation of Their Properties", PROPELLANTS, EXPLOSIVES, PYROTECHNICS., Bd. 28, Nr. 2, 1. April 2003 (2003-04-01), Seiten 77-82, XP055787162, DE ISSN: 0721-3115, DOI: 10.1002/prep.200390012

## Beschreibung

Die vorliegende Erfindung betrifft ein kontinuierliches Verfahren zur Synthese von 4,6-Dinitrobenzofuroxan.

Zur Herstellung von 4,6-Dinitrobenzofuroxan (4,6-DNBF) sind in der Literatur zwei Hauptwege und eine Kombination aus beiden Hauptwegen zu finden:

### Syntheseroute 1: Thermische Zersetzung von Pikrvlazid

Eine Syntheseroute umfasst die thermische Zersetzung von Pikrylazid. Hierbei wird 2,4,6-Trinitrochlorbenzen (Pikrylchlorid) mit Natriumazid (NaN₃) zu 2,4,6-Trinitroazidobenzen (Pikrylazid) umgesetzt. Anschließend erfolgt ein Ringschluss der Nitrogruppe mit der benachbarten Azidogruppe durch Thermolyse in einem höhersiedenden Lösungsmittel, z.B. Essigsäure, Toluen oder Xylen, zu einem Furoxan-Ring, wodurch 4,6-Dinitrobenzofuroxan entsteht *(*Ernst Schrader: Ber. Dtsch. Chem. Ges. 50, 777 (1917*);* P. A. S. Smith, J. H. Boyer; Org. Syn. 31, 14 (1951*);* Org. Syn., Coll. Vol. 4, 74 (1963); Vol. 37, 1 (1957*)).*

Nach einer Synthesevariante von Korczynski und Namylslowski lassen sich Pikrylchlorid und Natriumazid in Eisessig bei 90 °C in einer Eintopfreaktion zu 4,6-DNBF umsetzen, ohne die empfindliche explosive Zwischenstufe Pikrylazid isolieren zu müssen *(*A. Korczynski, St. Namylslowski, Bull. Soc. Chim. 35, 1186 (1924*)).* Spear et al. und Agrawal et al. haben diese Variante später angewendet, um Gramm-Mengen von 4,6-DNBF zu synthetisieren *(*Robert J. Spear, William P. Norris, Roger W. Read, MRL-TN-470 (1983*);* J. P. Agrawal, Mehilal, R. B. Salunke, P. D. Shinde, Propell. Explos. Pyrotech., 2003, 28, 77*).*

### Syntheseroute 2: Nitrierung von Benzofuroxan

Daneben hat Drost 1899 als zweite Syntheseroute die direkte Nitrierung von Benzofuroxan mit konzentrierter Schwefelsäure und Salpetersäure innerhalb des Temperaturbereiches 0 °C bis 5 °C und bei 40 °C beschrieben. Aufgrund der Unkenntnis der richtigen heterozyklischen Struktur, die erst in den 1950er Jahren aufgeklärt werden konnte, hat Drost diese Verbindung als o-Dinitrosobenzol bzw. die nitrierte Form als "Nitroderivate des o-Dinitrosobenzols" bezeichnet. Bei dieser Präparationsmethode wird Benzofuroxan unter Kühlen in kalter, konzentrierter Schwefelsäure gelöst, in Schwefelsäure gelöstes Kaliumnitrat oder in Schwefelsäure gelöste Salpetersäure unter Kühlen zugegeben und das Gemisch auf 40 °C erwärmt. Danach lässt man auf Raumtemperatur abkühlen und gießt zum Ausfällen des 4,6-DNBF auf Eiswasser *(*P. Drost, Liebigs Ann, Chem., B. 370, 49-69 (1899*)).*

Green und Rowe beschrieben ein sehr ähnliches, auf Schwefelsäure basierendes Verfahren, wobei sie durch angepasste Stöchiometrie neben 4,6-Dinitrobenzofuroxan auch 6-Nitrobenzofuroxan synthetisieren *(*A. G, Green, F. M. Rowe, J. Chem. Soc. 103, 2023 (1913*);* J. Chem. Soc. 113, 67 (1918*)).*

Spear et al. beschreiben ein auf Drost beruhendes Verfahren mit leichten Modifikationen, wonach Benzofuroxan in warmer Schwefelsäure gelöst und in Eis wieder abgekühlt wird. Eine Mischung aus Schwefel- und Salpetersäure wird portionsweise zugegeben, wobei die Temperatur unter 25 °C gehalten wird. Dann wird auf 40 °C erwärmt, 5 Minuten bei dieser Temperatur gehalten und auf Eis geschüttet. Das rohe 4,6-DNBF wird aus Chloroform umkristallisiert. Die Ausbeute ist mit knapp 55 % angegeben *(*Robert J. Spear, William P. Norris, Roger W. Read, MRL-TN-470 (1983*)).*

Zbarsky, Stepashkow und Yudin beschreiben die Nitrierung von Benzofuroxan und Mononitrobenzofuroxanderivaten mit Salpetersäure alleine und mit Mischsäure in einem Temperaturbereich von -10 bis 0 °C:
i. 4-Nitrobenzofuroxan (4-NBF) wird in 98%iger Salpetersäure bei Raumtemperatur über 2 Wochen gerührt. Die Ausbeute zum 4,6-DNBF wird mit 60 % angegeben und der Umsatz als nicht vollständig bezeichnet.
ii. 6-Nitrobenzofuroxan (6-NBF) wird mit 98%iger Salpetersäure bei Raumtemperatur zum 4,6-DNBF nitriert. Die Reaktion ist innerhalb von 24 Stunden abgeschlossen. Nach Waschen mit kaltem Wasser beträgt die Ausbeute 50 %. Die Nitrierung mit Mischsäure ergibt nach Waschen mit Wasser eine Ausbeute von 52 bis 55 %.
iii. Benzofuroxan wird mit 98%iger Salpetersäure nitriert, indem es langsam zu -10 °C kalter Salpetersäure dosiert und die Temperatur unter 0 °C gehalten wird. Nach einer unbestimmten Rührzeit wird die Mischung auf Eis geschüttet, abfiltriert und mit kaltem Wasser gewaschen.
iv. Benzofuroxan wird mittels Mischsäure nitriert, indem es zwischen -10 °C und -5 °C in Schwefelsäure gelöst wird. Anschließend gibt man Salpetersäure oder Mischsäure dazu. Das Produkt wird analog iii. isoliert. Die Ausbeute wird mit max. 77 % angegeben. *(*V. L. Zbarsky, D. M. Stepashkow, N. V. Yudin, new trends in research of energetic materials (ntrem), 704-707 (2004*))*

### Syntheseroute 3: Kombination aus Azidierung und Nitrierung

Bailey und Gase beschreiben die Azidierung von 2,4-Dinitrochlorbenzen mit Natriumazid zu 2,4-Dinitroazidobenzen, die anschließende Nitrierung zu 2,4,6-Trinitroazidobenzen und die thermische Zersetzung unter Ringschluss zu 4,6-DNBF *(*A. S. Bailey, J. R. Case, Tetrahedron, 113-131 (1958*)).*

Als weiterer Stand der Technik können Dong Yan: "Preparation of 5,7-diamino-4,6-dinitrobenzenfuroxan", Baopo Qicai, 1.Januar 2013, Seiten 10.13, XP055785914 und MacCormack P. et al: "Unusual structure in Meisenheimer complex formation from the highly electrophilic 4,6-dinitrobenzofuroxan", J. Org. Chem., Vol. 53, No. 18, 1988, Seiten 4407-4409 genannt werden.

### Gegenstand der Erfindung

Problematisch ist bei allen bekannten Syntheserouten, dass hochgefährliche, explosive und/oder giftige Verbindungen entstehen oder eingesetzt werden. Diese Tatsache steht der großtechnischen Herstellung von 4,6-Dinitrobenzofuroxan im Wege bzw. erschwert diese deutlich.

Der vorliegenden Erfindung liegt daher die Aufgabe zu Grunde, ein Verfahren bereitzustellen, mit welchem in kurzer Zeit große Mengen, z.B. pro Tag Mengen im zweistelligen Kilogrammbereich, 4,6-Dinitrobenzofuroxan hergestellt werden können. Das Verfahren soll möglichst einfach durchführbar sein und insbesondere wenige Schritte umfassen. Die Ausbeute soll aus ökonomischen und ökologischen Gründen ausreichend hoch sein. Eine weitere Aufgabe besteht darin, ein Verfahren bereitzustellen, das ein reines Produkt ergibt, wodurch die Aufarbeitung des Produktes vereinfacht wird. Insbesondere soll das Produkt zur weiteren Umsetzung nicht umkristallisiert werden müssen. Verunreinigungen, insbesondere die isomere Verbindung 5,6-Dinitrobenzofuroxan, sollen in möglichst geringen Mengen entstehen. Vorzugsweise ist auch die Aufreinigung verbrauchter Substanzen einfach und/oder ein Teil der Lösungen ist wiederverwendbar. Die Bildung organischer Lösungsmittelabfälle und toxischer Gase soll vermieden werden. Sehr hohe Temperaturen über lange Zeiten, z.B. ein Erhitzen unter Rückfluss über mehrere Stunden, ist aus ökonomischen Gründen und Sicherheitsgründen möglichst zu vermeiden.

Mindestens eine dieser Aufgaben wird gelöst durch ein kontinuierliches Verfahren zur Synthese von 4,6-Dinitrobenzofuroxan, das bevorzugt die Nitrierung von Benzofuroxan und/oder 4-Nitrobenzofuroxan und/oder 6-Nitrobenzofuroxan in konzentrierter Salpetersäure bei einer Temperatur von 42 bis 90 °C umfasst, wobei die konzentrierte Salpetersäure 60 bis 100 Gew.-% Salpetersäure enthält und wobei zur Nitrierung keine Schwefelsäure eingesetzt wird.

Vorteilhaft ist, dass das Verfahren eine einstufige Synthese umfasst, wodurch ein einfaches Verfahren ermöglicht wird. Für die Nitrierung wird die im Stand der Technik zur Nitrierung von Benzofuroxan verwendete Temperatur deutlich angehoben. Die höhere Reaktionstemperatur führt zu einer höheren Reaktionsgeschwindigkeit, wodurch ein kontinuierliches Verfahren erst möglich wird. Das erfindungsgemäße kontinuierliche Verfahren weist gegenüber bekannten Batch-Verfahren eine erhöhte Produktivität auf. Die kontinuierliche Prozessführung führt ferner dazu, dass keine sehr großen Stoffmengen an einem singulären Ort vorliegen. Eine solche Konzentrierung stellt bei den Edukten, Zwischenprodukten und/oder Endprodukten der Synthese von 4,6-Dinitrobenzofuroxan ein Risiko dar. Wegen der räumlichen und zeitlichen Verteilung der Stoffmengen ist die deutlich erhöhte Synthesetemperatur weniger kritisch. Mit anderen Worten ermöglicht die höhere Reaktionstemperatur eine kontinuierliche Verfahrensführung und die kontinuierliche Verfahrensführung erlaubt die höhere Reaktionstemperatur. Durch die kontinuierliche Prozessführung lassen sich sicher und ökonomisch Bulkmengen hoher Reinheit herstellen. Überraschenderweise wird durch das erfindungsgemäße Verfahren außerdem ein reineres Produkt erhalten. Trotz der erhöhten Temperatur wird über die Bildung der isomeren Verunreinigung 5,6-Dinitrobenzofuroxan verhindert. Als alleiniges Nitrieragens wird hochkonzentrierte Salpetersäure verwendet. Auf die Verwendung von Schwefelsäure oder anderer Mischsäuren, z.B. einem Gemisch aus Salpetersäure und Essigsäureanhydrid, kann verzichtet werden. Auch durch die Verwendung reiner Salpetersäure als Nitrieragens wird die Bildung der isomeren Verunreinigung 5,6-Dinitrobenzofuroxan unterdrückt. Eine spezielle Aufreinigung, insbesondere eine Umkristallisation, ist ebenfalls nicht nötig.

Bevorzugt wird in dem Verfahren als Edukt (der Nitrierung) Benzofuroxan eingesetzt. In bestimmten Ausführungsformen des Verfahrens enthält das Edukt Benzofuroxan sowie 4-Nitrobenzofuroxan und/oder 6-Nitrobenzofuroxan. In bestimmten Ausführungsformen des Verfahrens wird 4-Nitrobenzofuroxan und/oder 6-Nitrobenzofuroxan als Edukt eingesetzt. In bestimmten Ausführungsformen des Verfahrens wird ein Gemisch von Benzofuroxan, 4-Nitrobenzofuroxan und 6-Nitrobenzofuroxan als Edukt eingesetzt.

Ein kontinuierliches Verfahren steht insbesondere im Kontrast zu sogenannten "Batch-Verfahren".

Der Ausdruck "in konzentrierter Salpetersäure" soll zum Ausdruck bringen, dass während der Synthese (im Wesentlichen alleine) Salpetersäure das Nitrieragens und Lösungsmittel darstellt. Nicht ganz ausgeschlossen wird durch diesen Wortlaut, dass die konzentrierte Salpetersäure Lösungsmittel, insbesondere Wasser oder auch organische Lösungsmittel, enthält. Auch kann die konzentrierte Salpetersäure kleine Mengen an Essigsäureanhydrid, z.B. 0 bis 5 Gew.-% oder bis zu 10 Gew.-% Essigsäureanhydrid, enthalten, allerdings tragen diese dann nur unwesentlich zur Nitrierung bei.

Essigsäureanhydrid trägt insbesondere dann wesentlich zur Nitrierung bei, wenn das Produkt der Synthese 5,6-Dinitrobenzofuroxan im einstelligen Gew.-%-Bereich (also mindestens 1 Gew.-%) enthält. In bevorzugten Ausführungsformen enthält die konzentrierte Salpetersäure (im Wesentlichen) nur Salpetersäure und optional Wasser. In bevorzugten Ausführungsformen besteht die konzentrierte Salpetersäure aus Salpetersäure, optional mit einem Anteil Wasser. In bevorzugten Ausführungsformen enthält die konzentrierte Salpetersäure 80 bis 100 Gew.-%, bevorzugt 90 bis 99 Gew.-%, besonders bevorzugt etwa 98 Gew.-%, Salpetersäure. Bevorzugt wird die Differenz in diesen Ausführungsformen (im Wesentlichen) aus Wasser gebildet.

Die Verben "enthalten" und "umfassen" und ihre Konjugationen umfassen das Verb "bestehen aus" und seine Konjugationen.

Weitere bevorzugte Ausführungsformen werden auch in den Unteransprüchen angegeben.

Die Merkmale und Ausführungsformen aus der Beschreibung können beliebig miteinander kombiniert werden, sofern sich nicht Gegenteiliges aus diesen ergibt.

In einer bevorzugten Ausführungsform wird (im Wesentlichen) kein bzw. weniger als 1 Gew.-% 5,6-Dinitrobenzofuroxan gebildet. Mit anderen Worten enthält das Produkt der Nitrierung kein bzw. weniger als 1 Gew.-% 5,6-Dinitrobenzofuroxan. Mit nochmals anderen Worten enthält das im Verfahren mittels Nitrierung gebildete 4,6-Dinitrobenzofuroxan kein bzw. weniger als 1 Gew.-% 5,6-Dinitrobenzofuroxan als Verunreinigung.

Eine wichtige Auswirkung der Verwendung reiner Salpetersäure ist, dass die isomere Verbindung 5,6-Dinitrobenzofuroxan nicht gebildet wird, bzw. dessen Gehalt im Produkt unterhalb der analytischen Nachweisgrenze liegt. Es wird ein reineres Produkt erhalten und weitere Prozessschritte zur Reinheitserhöhung, insbesondere ein Umkristallisieren, werden überflüssig.

In einer bevorzugten Ausführungsform wird zur Nitrierung (von Benzofuroxan und/ oder 4-Nitrobenzofuroxan und/oder 6-Nitrobenzofuroxan) kein Essigsäureanhydrid bzw. maximal 10 Gew.-% Essigsäureanhydrid eingesetzt. Bei der Nitrierung unter Verwendung von Schwefelsäure ist das gewünschte Hauptprodukt 4,6-Dinitrobenzofuroxan besonders stark, d.h. im einstelligen Prozentbereich, mit der isomeren Verbindung 5,6-Dinitrobenzofuroxan verunreinigt. Soll die Reinheit des 4,6-Dinitrobenzofuroxans dann erhöht werden, muss diese Kontamination 5,6-Dinitrobenzofuroxan durch einen weiteren Verfahrensschritt, z.B. eine Umkristallisation, abgetrennt werden. Als weiterer Vorteil eines solchen erfindungsgemäßen Verfahrens ist es ökonomischer, Salpetersäure zu entsorgen oder aufzubereiten als eine Mischsäure aus Salpetersäure und Schwefelsäure und/oder Essigsäureanhydrid.

In einer bevorzugten Ausführungsform umfasst das Verfahren zumindest einen der Schritte ausgewählt aus A), B1), B2), B3), B4) und C), bevorzugt zumindest die Schritte A), B1), B2) und C):
A) ein Initialisieren des Verfahrens durch Mischen von konzentrierter Salpetersäure und Benzofuroxan und/oder 4-Nitrobenzofuroxan und/oder 6-Nitrobenzofuroxan in einem ersten Reaktor, wobei ein Gemisch gebildet wird, wobei die konzentrierte Salpetersäure 60 bis 100 Gew.-% Salpetersäure enthält,
B) ein Fortführen des Verfahrens durch
B1) Zugeben weiterer Salpetersäure und weiteren Benzofuroxans und/oder 4-Nitrobenzofuroxans und/oder 6-Nitrobenzofuroxans, vorzugsweise in den ersten Reaktor und/oder das Gemisch, um weiteres Gemisch zu bilden, wobei die konzentrierte Salpetersäure 60 bis 100 Gew.-% Salpetersäure enthält und wobei die Temperatur in dem ersten Reaktor während B) auf 42 bis 90 °C eingestellt wird,
B2) Überführen von Gemisch, vorzugsweise aus dem ersten Reaktor, in einen zweiten Reaktor, wobei die Temperatur in dem zweiten Reaktor auf 42 bis 80 ° C eingestellt wird,
   gegebenenfalls B3) Überführen von Gemisch aus dem zweiten Reaktor in einen dritten Reaktor, wobei die Temperatur in dem dritten Reaktor auf 42 bis 80 °C eingestellt wird,
   gegebenenfalls B4) Überführen von Gemisch aus dem dritten Reaktor in einen vierten Reaktor, wobei die Temperatur in dem vierten Reaktor auf 42 bis 80 °C eingestellt wird, und
C) ein Überführen von Gemisch, vorzugsweise aus dem letzten Reaktor, in einen Fällbehälter, wobei der Fällbehälter bei einer Temperatur zwischen 0 und 25 °C Wasser oder eine wässrige Lösung mit bis zu 55 Gew.-%, bevorzugt 25 bis 45 Gew.-%, Salpetersäure enthält, wodurch ein Niederschlag ausgefällt wird, der 4,6-Dinitrobenzofuroxan enthält.

Dieses Verfahren ist besonders vorteilhaft, da es eine Ausfällung des Produktes umfasst. Vorzugsweise wird B) und C) kontinuierlich durchgeführt. Mit anderen Worten umfasst das Fortführen des Verfahrens vorzugsweise B) und C). Der in C) ausgefällte Niederschlag wird vorzugsweise, insbesondere periodisch, abgetrennt, insbesondere abgenutscht. Der abgetrennte/abgenutschte Niederschlag wird vorzugsweise mit Wasser gewaschen. Das zum Waschen verwendete Wasser wird vorzugsweise in den Fällbehälter geführt. Bei der Reaktion bzw. bei der Verdünnung (Fällung) freiwerdende nitrose Gase werden vorzugsweise durch einen Abgaswäscher aus der Abluft entfernt.

Bevorzugt wird die Temperatur in den Reaktoren eingestellt, indem die Kühlleistung des Kühlsystems der Reaktoren an die Geschwindigkeit der exothermen Reaktion (Nitrierung) angepasst wird. Die Geschwindigkeit der exothermen Reaktion ergibt sich insbesondere aus der Konzentration der Edukte und der Temperatur in den Reaktoren.

Vorzugsweise wird das Gemisch in jedem Reaktor gerührt. Vorzugsweise findet in jedem Reaktor die Nitrierung von Benzofuroxan und/oder 4-Nitrobenzofuroxan und/oder 6-Nitrobenzofuroxan zu 4,6-Dinitrobenzofuroxan statt.

Durch den Niederschlag kann sich in dem Fällbehälter eine Suspension bilden. Der Begriff "wässrige Lösung mit bis zu 55 Gew.-%, bevorzugt 25 bis 45 Gew.-%, Salpetersäure" umfasst dagegen nur die gelösten Moleküle. Mit anderen Worten ist die "wässrige Lösung mit bis zu 55 Gew.-%, bevorzugt 25 bis 45 Gew.-%, Salpetersäure" eine Flüssigkeit, die bis zu 55 Gew.-%, bevorzugt 25 bis 45 Gew.-%, Salpetersäure enthält, und in dem Fällbehälter vorliegt, gegebenenfalls neben suspendierten oder ausgefallenen Feststoffen.

Der "letzte Reaktor" ist der zweite Reaktor, wenn nur ein erster und zweiter Reaktor verwendet werden. Bei Verwendung eines dritten Reaktors (aber keines vierten Reaktors) ist der dritte Reaktor der letzte Reaktor. Bei Verwendung eines vierten Reaktors ist der vierte Reaktor der letzte Reaktor.

Vorzugsweise erfolgt A) über einen Zeitraum von 1 bis 6 Minuten. Mit anderen Worten wird das Gemisch im ersten Reaktor vorzugsweise 1 bis 6 Minuten gemischt, ehe weiteres Edukt zugegeben wird.

Vorzugsweise erfolgen B1) und B2) gleichzeitig. Vorzugsweise erfolgen B) und C) gleichzeitig.

Vorzugsweise wird Gemisch durch ein kontinuierliches Zugeben von Salpetersäure und Benzofuroxan und/oder 4-Nitrobenzofuroxan und/oder 6-Nitrobenzofuroxan in den ersten Reaktor und/oder das Gemisch aus dem ersten Reaktor verdrängt und über eine Rohrleitung in den zweiten Reaktor überführt.

Vorzugsweise wird auch das gegebenenfalls durchgeführte Überführen von Gemisch aus dem zweiten Reaktor in einen dritten Reaktor und das gegebenenfalls durchgeführte Überführen von Gemisch aus einem dritten Reaktor in einen vierten Reaktor und das Überführen von Gemisch aus dem letzten Reaktor in einen Fällbehälter durch ein kontinuierliches Zugeben von Salpetersäure und Benzofuroxan und/oder 4-Nitrobenzofuroxan und/oder 6-Nitrobenzofuroxan in den ersten Reaktor und/oder das Gemisch und ein Verdrängen des Gemisches ausgelöst. Vorzugsweise erfolgt das Überführen über mindestens eine Rohrleitung.

Vorzugsweise umfasst das Verfahren B3). Dies ist insbesondere dann der Fall, wenn in dem Gemisch bei Überführen in den dritten Reaktor durch eine längere Reaktionszeit noch weiteres 4,6-Dinitrobenzofuroxan gebildet werden kann. In einer bevorzugten Ausführungsform umfasst das Verfahren B3) und B4). Dies ist insbesondere dann der Fall, wenn in dem Gemisch bei Überführen in den vierten Reaktor durch eine längere Reaktionszeit noch weiteres 4,6-Dinitrobenzofuroxan gebildet werden kann. Vorzugsweise nimmt die Konzentration an Benzofuroxan in dem Gemisch ausgehend vom ersten Reaktor mit jedem Reaktor ab. Vorzugsweise nimmt die Temperatur des Gemisches ausgehend vom ersten Reaktor mit jedem Reaktor ab. Vorzugsweise erfolgt das Zugeben weiteren Benzofuroxans und/oder 4-Nitrobenzofuroxans und/oder 6-Nitrobenzofuroxans in B1) in den ersten Reaktor und/oder das Gemisch über eine Dosierschnecke.

In einer bevorzugten Ausführungsform wird die Temperatur in dem ersten Reaktor während B), insbesondere durch Kühlen, auf 42 bis 90 °C, bevorzugt auf 50 bis 80 °C, besonders bevorzugt auf 55 bis 72 °C, eingestellt.

Die eingestellte Temperatur ergibt sich aus der bei der Nitrierung freiwerdenden Wärme und der Kühlung des Reaktors. Bei dieser Temperatur ist die Durchführung des kontinuierlichen Verfahrens besonders günstig.

In einer bevorzugten Ausführungsform weist die Salpetersäure in A) eine Temperatur von 35 bis 90 °C, bevorzugt von 42 bis 75 °C, besonders bevorzugt von 45 bis 60 °C, ganz besonders bevorzugt von etwa 50 °C, auf. Diese Temperatur bezieht sich auf die Temperatur der Salpetersäure (unmittelbar) vor dem Mischen mit Benzofuroxan und/oder 4-Nitrobenzofuroxan und/oder 6-Nitrobenzofuroxan. Vorzugsweise wird die Salpetersäure in A) vorgelegt und Benzofuroxan und/oder 4-Nitrobenzofuroxan und/oder 6-Nitrobenzofuroxan wird in die Salpetersäure gegeben, insbesondere über eine Dosierschnecke.

So lässt sich das Verfahren besonders effektiv und risikolos starten. Durch das Mischen und die beginnende Reaktion wird Wärme frei, so dass die für B1) benötigte Mindesttemperatur schnell erreicht wird. Das Verfahren kann dann einfach in das kontinuierliche Verfahren übergehen.

In einer bevorzugten Ausführungsform wird die Temperatur in dem zweiten und/oder gegebenenfalls dritten und/oder gegebenenfalls vierten Reaktor auf 50 bis 72 °C, besonders bevorzugt auf 55 oder 58 bis 63 °C, eingestellt, insbesondere durch Kühlen. In dem zweiten und/oder gegebenenfalls dritten und/oder gegebenenfalls vierten Reaktor kann die Nitrierung von Benzofuroxan und/oder 4-Nitrobenzofuroxan und/oder 6-Nitrobenzofuroxan in dem Gemisch fortgeführt werden. Da die Konzentration unreagierten Edukts mit jedem Reaktor abnimmt, kommt es von selbst zunehmend zum Abkühlen des Gemisches, was für die nachfolgende Fällung hilfreich ist. Die Abkühlung kann durch ein Kühlen des jeweiligen Reaktors beschleunigt werden.

In einer bevorzugten Ausführungsform beträgt die durchschnittliche Verweilzeit des Gemisches bzw. eines Moleküls des Gemisches in jedem Reaktor 3 bis 30 Minuten, bevorzugt 5 bis 10 Minuten.

In dieser Zeit kann für das kontinuierliche Verfahren unter den zuvor genannten Bedingungen eine optimale Ausbeute erzielt werden. Die durchschnittliche Verweilzeit ergibt sich aus der kontinuierlichen Fließgeschwindigkeit der Salpetersäure und des Volumens des jeweiligen Reaktors bzw. der Reaktorkaskade.

In einer bevorzugten Ausführungsform wird das Gemisch zwischen dem letzten Reaktor und C) abgekühlt, vorzugsweise durch mindestens einen weiteren Reaktor mit Kühlung und/oder mindestens einen Rohrbündeltauscher, vorzugsweise auf eine Temperatur von 35 bis 15 °C, besonders bevorzugt auf eine Temperatur von 25 bis 10 °C.

Nach weiterem Kühlen bzw. mit der so erreichten Temperatur kann bei der nachfolgenden Fällung ein optimales Ergebnis erzielt werden.

In einer bevorzugten Ausführungsform ist/wird die mittlere Korngröße bzw. Partikelgröße des 4,6-Dinitrobenzofuroxans bzw. des in C) ausgefällten Niederschlages über die Temperatur des Wassers oder der wässrigen Lösung in dem Fällbehälter einstellbar oder eingestellt.

In einer bevorzugten Ausführungsform ist/wird
i) durch eine Temperatur zwischen 10 und 15 °C eine mittlere Korngröße bzw. Partikelgröße von 100 bis 150 µm und/oder
ii) durch eine Temperatur unter 10 °C eine mittlere Korngröße bzw. Partikelgröße von unter 100 µm und/oder
ii) durch eine Temperatur von 15 bis 25 °C, bevorzugt um etwa 20 °C, eine mittlere Korngröße bzw. Partikelgröße von 150 bis 200 µm,
   einstellbar oder eingestellt.

In einer besonders bevorzugten Ausführungsform ist/wird durch eine Temperatur zwischen 10 und 15 °C eine mittlere Korngröße bzw. Partikelgröße von 100 bis 150 µm einstellbar oder eingestellt.

Die Temperatur in dem Fällbehälter ergibt sich aus der Wärme des Gemisches, aus freiwerdender Wärme durch Verdünnung des Gemisches und der Temperatur des Wassers bzw. der wässrigen Lösung. Die Temperatur in dem Fällbehälter kann auch durch eine Kühlung des Fällbehälters und die Austauschrate der Flüssigkeit in dem Behälter gesteuert werden. Das Verfahren kann somit derart gesteuert werden, dass das Produkt optimal für die weitere Verwendung geeignet ist, ohne dass weitere Zwischenschritte wie Mahlen, Granulieren etc. erforderlich sind, um die mittlere Korngröße bzw. Partikelgröße einzustellen. Somit wird das Verfahren weiter vereinfacht.

In einer bevorzugten Ausführungsform umfasst das Verfahren also ein Ausfällen eines Niederschlages, wobei der Niederschlag 4,6-Dinitrobenzofuroxan enthält und wobei das Ausfällen vorzugsweise durch Verdünnen der konzentrierten Salpetersäure mit Wasser oder wässriger Lösung mit bis zu 55 Gew.-%, bevorzugt 25 bis 45 Gew.-%, Salpetersäure erfolgt. Vorzugsweise ist/wird die mittlere Korngröße bzw. Partikelgröße des 4,6-Dinitrobenzofuroxans bzw. des Niederschlages über die Temperatur des Wassers oder der wässrigen Lösung einstellbar oder eingestellt. Vorzugsweise ist/wird
i) durch eine Temperatur zwischen 10 und 15 °C eine mittlere Korngröße bzw. Partikelgröße von 100 bis 150 µm und/oder
ii) durch eine Temperatur unter 10 °C eine mittlere Korngröße bzw. Partikelgröße von unter 100 µm und/oder
ii) durch eine Temperatur von 15 bis 25 °C, bevorzugt um etwa 20 °C, eine mittlere Korngröße bzw. Partikelgröße von 150 bis 200 µm,
einstellbar oder eingestellt. In einer besonders bevorzugten Ausführungsform ist/wird durch eine Temperatur zwischen 10 und 15 °C eine mittlere Korngröße bzw. Partikelgröße von 100 bis 150 µm einstellbar oder eingestellt.

In einer bevorzugten Ausführungsform wird das Gemisch vor dem Ausfällen abgekühlt, vorzugsweise auf eine Temperatur von 35 bis 15 °C, besonders bevorzugt auf eine Temperatur von 25 bis 10 °C.

Die mittlere Korngröße bzw. Partikelgröße wird im Rahmen der Erfindung mittels Laserstreuung auf einem Malvern Mastersizer 2000 gemessen und als D50-Wert angegeben.

In einer bevorzugten Ausführungsform enthält der Niederschlag bzw. das Produkt des Verfahrens bzw. das im Verfahren gebildete 4,6-Dinitrobenzofuroxan mindestens 97 Gew.-%, bevorzugt 98 bis 100 Gew.-%, 4,6-Dinitrobenzofuroxan.

Mit anderen Worten ist das Produkt bzw. der Niederschlag bzw. das 4,6-Dinitrobenzofuroxan besonders rein.

In einer bevorzugten Ausführungsform enthält der Niederschlag bzw. das Produkt des Verfahrens bzw. das im Verfahren gebildete 4,6-Dinitrobenzofuroxan weniger als 4 Gew.-%, bevorzugt weniger als 2 Gew.-%, besonders bevorzugt weniger als 1 Gew.-%, 5,6-Dinitrobenzofuroxan.

In einer bevorzugten Ausführungsform enthält der Niederschlag bzw. das Produkt des Verfahrens bzw. das im Verfahren gebildete 4,6-Dinitrobenzofuroxan weniger als 1 Gew.-% 6-Nitrobenzofuroxan.

In einer bevorzugten Ausführungsform enthält der Niederschlag bzw. das Produkt des Verfahrens bzw. das im Verfahren gebildete 4,6-Dinitrobenzofuroxan weniger als 1 Gew.-% 4-Nitrobenzofuroxan.

In einer bevorzugten Ausführungsform werden der Nitrierung bzw. dem ersten Reaktor und/oder dem Gemisch pro Stunde pro kg Benzofuroxan (und/oder 4-Nitrobenzofuroxan und/oder 6-Nitrobenzofuroxan) 6 bis 12 L Salpetersäure (berechnet auf 98 Gew.-%ige Salpetersäure) zugeführt. Bevorzugt werden dem Fällbehälter pro Stunde pro kg Benzofuroxan (und/oder 4-Nitrobenzofuroxan und/oder 6-Nitrobenzofuroxan) 14 bis 25 L Wasser zugeführt bzw. bevorzugt werden in dem Fällbehälter pro Stunde pro kg Benzofuroxan (und/oder 4-Nitrobenzofuroxan und/oder 6-Nitrobenzofuroxan) 14 bis 25 L Wasser oder eine wässrige Lösung mit bis zu 55 Gew.-%, bevorzugt 25 bis 45 Gew.-%, Salpetersäure ersetzt.

Die Angabe pro kg Benzofuroxan (und/oder 4-Nitrobenzofuroxan und/oder 6-Nitrobenzofuroxan) bezieht sich auf 1 kg Edukt, das ein Benzofuroxangrundgerüst aufweist.

In einer bevorzugten Ausführungsform wird das Benzofuroxan und/oder 4-Nitrobenzofuroxan und/oder 6-Nitrobenzofuroxan als Pulver oder Granulat in den ersten Reaktor und/oder das Gemisch gegeben.

In einer bevorzugten Ausführungsform weisen Salpetersäure und Benzofuroxan und/ oder 4-Nitrobenzofuroxan und/oder 6-Nitrobenzofuroxan in B1) (unmittelbar) vor Zugabe in den ersten Reaktor und/oder das Gemisch eine Temperatur zwischen 10 und 40 °C, bevorzugt zwischen 15 und 30 °C, auf.

In einer bevorzugten Ausführungsform weist das Verfahren eine stöchiometrische Ausbeute von mindestens 44 %, vorzugsweise 35 bis 80 % auf.

In einer bevorzugten Ausführungsform ist ein Umkristallisieren des Niederschlages bzw. des Produktes des Verfahrens bzw. des im Verfahren gebildeten 4,6-Dinitrobenzofuroxans nicht erforderlich.

Mit anderen Worten kann das im Verfahren gebildete 4,6-Dinitrobenzofuroxan ohne weitere Aufreinigung zur Herstellung von (An-)Zündstoffen (z. B. KDNBF, K-HA-DNBF, CsDNBF, RbDNBF) oder zur Herstellung von vom DNBF-abgeleiteten Heterozyklen (z.B. 5,7-Diamino-4,6-dinitro-benzofuroxan) verwendet werden.

In einer bevorzugten Ausführungsform umfasst das Verfahren keine Umkristallisation des Niederschlages bzw. des Produktes des Verfahrens bzw. des im Verfahren gebildeten 4,6-Dinitrobenzofuroxans.

In einer bevorzugten Ausführungsform wird in dem Verfahren keine Schwefelsäure und kein Essigsäureanhydrid verwendet.

In einer bevorzugten Ausführungsform wird in dem Verfahren kein Pikrylchlorid und/ oder 2,4-Dinitrochlorbenzol eingesetzt.

In einer bevorzugten Ausführungsform wird in dem Verfahren kein Azid, insbesondere kein Natriumazid, eingesetzt.

In einer bevorzugten Ausführungsform wird in dem Verfahren kein organisches Lösungsmittel eingesetzt.

In einer bevorzugten Ausführungsform wird in dem Verfahren kein 2,4-Dinitroazidobenzen und/oder kein 2,4,6-Trinitroazidobenzen und/oder keine Stickstoffwasserstoffsäure gebildet.

Gegenstand der Erfindung ist auch der Niederschlag, erhältlich durch das erfindungsgemäße Verfahren.

Gegenstand der Erfindung ist auch 4,6-Dinitrobenzofuroxan, erhältlich durch das erfindungsgemäße Verfahren.

Gegenstand der Erfindung ist auch eine Vorrichtung zur Durchführung des erfindungsgemäßen Verfahrens.

Im Folgenden wird die Erfindung anhand eines Beispieles beschrieben, das nicht beschränkend sein soll:

### Beispiel für die prozesstechnische Auslegung

In diesem Beispiel erfolgt die Synthese als kontinuierliches Verfahren innerhalb einer Kaskade, umfassend acht Glasreaktoren mit je 12 L Nutzvolumen und Mantelkühlung und einem gekühlten Fällbehälter mit Rührwerk. Alle Reaktoren sind mit Druckluftrührern und Temperatursensoren ausgestattet. Von einem Tagesbehälter wird die Nitrierkaskade mit Salpetersäure nach geodätischem Prinzip versorgt. Zum Starten der Reaktion werden 4 bis 10 Liter der 98-%igen Salpetersäure in einem zwischengeschalteten Glasbehälter auf ca. 55 bis 60 °C vorgeheizt und in den ersten Reaktor überführt. 150 bis 300 g pulverförmiges oder granuliertes Benzofuroxan werden dabei über eine Dosierschnecke unter Rühren in den ersten Reaktor überführt, in dem die Salpetersäure mit einer Temperatur von ca. 50 °C vorgelegt ist. Nach dem Anspringen der Reaktion werden Benzofuroxan und 98-%ige Salpetersäure, welche eine Temperatur von ca. 15 °C bis 30 °C haben, weiter zudosiert. Die Temperatur wird im ersten Reaktor durch Intensivkühlung auf ca. 55 bis 72 °C gehalten. Ein Absinken der Temperatur auf unter 45 °C sollte vermieden werden, weil dadurch die Reaktion zum Stillstand kommen oder verzögert werden kann. Die Kinetik der Nitrierung ist dabei so schnell, dass die chemische Reaktion beim 3., spätestens beim 4. Reaktor, bereits beendet ist. Die nachfolgenden Reaktoren in der bestehenden Mehrzweckanlage dienen dazu, die DNBF-haltige Salpetersäure weiter abzukühlen. Die Säure verlässt die Kaskade mit einer Temperatur von 19 bis 30 °C und fließt in einen gerührten, stark gekühlten Fällbehälter. In diesem sind rund 120 L kaltes Wasser vorgelegt und weiteres Kaltwasser (ca. 5 bis 10 °C) wird in dem Maße zudosiert, wie die DNBF-Suspension zuläuft. Dabei wird die Salpetersäure auf ca. 25 bis 45 % verdünnt, wodurch DNBF ausfällt. Die Temperatur im intensiv gekühlten Fällbehälter liegt 5 bis 20 °C. Er wird soweit geleert, dass ca. 2/3 der Suspension auf eine oder mehrere Nutschen gepumpt werden. Durch die partielle Entleerung ergibt sich über die ganze Schicht ein gleichbleibendes, relativ grobes Korn. Die abgetrennte verdünnte Salpetersäure wird separat gesammelt und einer Säurebehandlung bzw. Entsorgung zugeführt. Der Filterkuchen wird durch Absaugen mit kaltem Wasser mehrmals gewaschen und portionsweise in Fässer abgefüllt. Das Waschwasser wird aufgefangen und wieder als Verdünnungswasser eingesetzt. Bei der Reaktion bzw. bei der Verdünnung freiwerdende nitrose Gase werden durch einen Abgaswäscher aus der Abluft entfernt.

### Dosierungen:

| | |
|---|---|
| Salpetersäure (ca. 98%ig): | 80 bis 140 l/h = 120 bis 210 kg/h |
| Benzofuroxan (BFO): | 5 bis 20 kg/h |
| Fällwasser: | 200 bis 300 l/h |

### Ausbeute:

Bei einer Reaktorlaufzeit von ca. 6 Stunden pro Arbeitstag (1-schichtiger Betrieb) ergibt sich eine Nettoexplosivstoffmenge von rund 50 bis 60 kg DNBF pro Arbeitstag. Dies entspricht einer stöchiometrischen Ausbeute von ca. 44 %. Ein gewisser Anteil bleibt in der Restsäure zurück und kann nicht mitgefällt werden, der größere Anteil wird wahrscheinlich von der Salpetersäure zu gasförmigen Nebenprodukten oxidiert.

### Fällkessel (Fällbehälter)

Durch unterschiedliche Temperatur- und Verdünnungsprofile im Fällkessel lassen sich verschiedene Körnungen produzieren. Allgemein ist die Körnung, dargestellt als mittlere Korngröße einer Verteilung, umso kleiner, je geringer die Temperatur im Fällkessel und je geringer die Konzentration an 4,6-Dinitrobenzofuroxan ist. Unter diesen Umständen wachsen die sich bildenden Kristalle weniger schnell als im Falle höherer Fälltemperatur und höherer Konzentration.

Bei einer Fälltemperatur im Bereich von 10 bis 15 °C erhält man eine mittlere Partikelgröße (sog. D50-Wert in der Laserbeugung) von etwa 100 bis 150 µm. Bei Fälltemperaturen zwischen 5 und 10 °C liegt die mittlere Partikelgröße deutlich unter 100 µm, bei Temperaturen um 20 °C beträgt die mittlere Korngröße bis zu 200 µm.

### Weiteres zu den Reaktoren

Durch die Auslegung als 8-Reaktoren-Kaskade ergibt sich bei den genannten Dosierleistungen und der nutzbaren Kühlleistung (Ergebnis der Leistung und Auslegung der Kühlanlage und des Reaktordesigns) folgende typische Temperaturverteilung in den 8 Reaktoren:
1) Reaktor 1: 55 bis 72°C
2) Reaktor 2: 50 bis 72°C
3) Reaktor 3: 50 bis 72°C
4) Reaktor 4: 50 bis 72°C
5) Reaktor 5: 30 bis 50°C
6) Reaktor 6: 15 bis 30°C
7) Reaktor 7: 10 bis 25°C
8) Reaktor 8: 10 bis 25°C
9) Fällbehälter: 0 bis 25 °C

Die Verwendung von 8 Reaktoren in einer Kaskade ist zur kontinuierlichen Nitrierung von Benzofuroxan bei höheren Temperaturen nicht zwingend notwendig. Die hohe Reaktionsgeschwindigkeit erfordert nur eine relativ kurze Verweilzeit der Edukte in den Reaktoren. Bei den zur Anwendung kommenden Dosierraten ist die Reaktion beim 3., spätestens jedoch beim 4. Reaktor beendet. Es lassen sich daher auch Designs mit nur 2 bis 4 Reaktoren realisieren. Die weiteren Reaktoren, die in dieser Anlage nur der Kühlung der Reaktionslösung dienen, lassen sich demnach auch durch andere technische Kühleinrichtungen substituieren, z.B. durch einen Rohrbündelwärmetauscher.

Denkbar ist, dass in nachfolgende Reaktoren (z.B. 2 und 3) noch zusätzlich Edukt (Benzofuroxan) dosiert werden. Jedoch würde man einen oder zwei weitere Reaktoren oder Kühlmittel zum Herunterkühlen der heißen Reaktionslösung benötigen.

Das prozesstechnische Design der Nitrierkaskade lässt sich hochskalieren. Es ist nur dafür zu sorgen, dass die Kühlleistung hoch genug ist, die Wärmemengen abzuführen, welche bei der Nitrierung und der Verdünnung der hochkonzentrierten Salpetersäure entstehen. Dies kann durch unterschiedliche technische Maßnahmen erzielt werden. Die Größe und Anzahl der Reaktoren richtet sich nach der gewünschten Leistung der Anlage und ist auf die Geschwindigkeit der Nitrierungsreaktion und die Abkühlung der DNBF-haltigen Salpetersäure anzupassen.

## Patentansprüche

1. Kontinuierliches Verfahren zur Synthese von 4,6-Dinitrobenzofuroxan, umfassend die Nitrierung von Benzofuroxan und/oder 4-Nitrobenzofuroxan und/oder 6-Nitrobenzofuroxan in konzentrierter Salpetersäure bei einer Temperatur von 42 bis 90 °C, wobei die konzentrierte Salpetersäure 60 bis 100 Gew.-% Salpetersäure enthält, **dadurch gekennzeichnet, dass** zur Nitrierung keine Schwefelsäure eingesetzt wird.

2. Verfahren nach Anspruch 1, **dadurch gekennzeichnet, dass** zur Nitrierung kein Essigsäureanhydrid eingesetzt wird.

3. Verfahren nach Anspruch 1 oder 2, wobei das Verfahren zumindest einen der Schritte ausgewählt aus A), B1), B2), B3), B4) und C), bevorzugt zumindest die Schritte A), B1), B2) und C), umfasst:
A) ein Initialisieren des Verfahrens durch Mischen von konzentrierter Salpetersäure und Benzofuroxan und/oder 4-Nitrobenzofuroxan und/oder 6-Nitrobenzofuroxan in einem ersten Reaktor, wobei ein Gemisch gebildet wird, wobei die konzentrierte Salpetersäure 60 bis 100 Gew.-% Salpetersäure enthält,
B) ein Fortführen des Verfahrens durch
B1) Zugeben weiterer Salpetersäure und Benzofuroxan und/oder 4-Nitrobenzofuroxan und/oder 6-Nitrobenzofuroxan, vorzugsweise in den ersten Reaktor und/oder das Gemisch, um weiteres Gemisch zu bilden, wobei die konzentrierte Salpetersäure 60 bis 100 Gew.-% Salpetersäure enthält und wobei die Temperatur in dem ersten Reaktor während B) auf 42 bis 90 °C eingestellt wird,
B2) Überführen von Gemisch, vorzugsweise aus dem ersten Reaktor, in einen zweiten Reaktor, wobei die Temperatur in dem zweiten Reaktor auf 42 bis 80 °C eingestellt wird,
gegebenenfalls B3) Überführen von Gemisch aus dem zweiten Reaktor in einen dritten Reaktor, wobei die Temperatur in dem dritten Reaktor auf 42 bis 80 °C eingestellt wird,
gegebenenfalls B4) Überführen von Gemisch aus dem dritten Reaktor in einen vierten Reaktor, wobei die Temperatur in dem vierten Reaktor auf 42 bis 80 °C eingestellt wird, und
C) ein Überführen von Gemisch, vorzugsweise aus dem letzten Reaktor, in einen Fällbehälter, wobei der Fällbehälter bei einer Temperatur zwischen 0 und 25 °C Wasser oder eine wässrige Lösung mit bis zu 55 Gew.-%, bevorzugt 25 bis 45 Gew.-%, Salpetersäure enthält, wodurch ein Niederschlag ausgefällt wird, der 4,6-Dinitrobenzofuroxan enthält.

4. Verfahren zumindest nach Anspruch 3, **dadurch gekennzeichnet, dass** die Temperatur in dem ersten Reaktor während B), insbesondere durch Kühlen, auf 45 bis 85 °C, bevorzugt auf 50 bis 80 °C, besonders bevorzugt auf 55 bis 72 °C, eingestellt wird.

5. Verfahren zumindest nach einem der Ansprüche 3 oder 4, **dadurch gekennzeichnet, dass** die Temperatur in dem zweiten und/oder gegebenenfalls dritten und/oder gegebenenfalls vierten Reaktor auf 50 bis 72 °C, besonders bevorzugt auf 55 bis 63 °C, eingestellt wird, insbesondere durch Kühlen.

6. Verfahren zumindest nach einem der Ansprüche 3 bis 5, **dadurch gekennzeichnet, dass** die durchschnittliche Verweilzeit des Gemisches bzw. eines Moleküls des Gemisches in jedem Reaktor 3 bis 30 Minuten, bevorzugt 4 bis 10 Minuten, beträgt.

7. Verfahren zumindest nach einem der Ansprüche 3 bis 6, **dadurch gekennzeichnet, dass** das Gemisch zwischen dem letzten Reaktor und C) abgekühlt wird, vorzugsweise durch mindestens einen weiteren Reaktor mit Kühlung und/oder mindestens einen Rohrbündeltauscher, vorzugsweise auf eine Temperatur von 35 bis 5 °C, bevorzugt von 25 bis 10 °C.

8. Verfahren zumindest nach einem der Ansprüche 1 bis 7, **dadurch gekennzeichnet, dass** das Verfahren ein Ausfällen eines Niederschlages umfasst, wobei der Niederschlag 4,6-Dinitrobenzofuroxan enthält, wobei das Ausfällen durch Verdünnen der konzentrierten Salpetersäure mit Wasser oder wässriger Lösung mit bis zu 55 Gew.-%, bevorzugt 25 bis 45 Gew.-%, Salpetersäure erfolgt, und wobei die mittlere Korngröße bzw. Partikelgröße des 4,6-Dinitrobenzofuroxans bzw. des Niederschlages über die Temperatur des Wassers oder der wässrigen Lösung eingestellt wird.

9. Verfahren zumindest nach Anspruch 8, **dadurch gekennzeichnet, dass**
i) durch eine Temperatur zwischen 10 und 15 °C eine mittlere Korngröße bzw. Partikelgröße von 100 bis 150 µm oder
ii) durch eine Temperatur unter 10 °C eine mittlere Korngröße bzw. Partikelgröße von unter 100 µm oder
iii) durch eine Temperatur von 15 bis 25 °C, bevorzugt um etwa 20 °C, eine mittlere Korngröße bzw. Partikelgröße von 150 bis 200 µm
eingestellt wird.

## Claims

1. Continuous process for the synthesis of 4,6-dinitrobenzofuroxane comprising nitration of benzofuroxane and/or 4-nitrobenzofuroxane and/or 6-nitrobenzofuroxane in concentrated nitric acid at a temperature of 42 to 90°C, wherein the concentrated nitric acid contains 60 to 100% by weight nitric acid, **characterised in that** no sulphuric acid is used for nitration.

2. Process according to claim 1, **characterized in that** no acetic anhydride is used for nitration.

3. Process according to claim 1 or 2, wherein the process comprises at least one of the steps selected from A), B1), B2), B3), B4) and C), preferably at least the steps A), B1), B2) and C):
A) an initialisation of the process by mixing concentrated nitric acid and benzofuroxane and/or 4-nitrobenzofuroxane and/or 6-nitrobenzofuroxane in a first reactor, whereby a mixture is formed, wherein the concentrated nitric acid contains 60 to 100% by weight of nitric acid,
B) a continuation of the process by
B1) adding further nitric acid and benzofuroxane and/or 4-nitrobenzofuroxane and/or 6-nitrobenzofuroxane, preferably into the first reactor and/or the mixture, to form further mixture, wherein the concentrated nitric acid contains 60 to 100 wt.% nitric acid and wherein the temperature in the first reactor during B) is adjusted to 42 to 90°C,
B2) transferring of mixture, preferably from the first reactor, to a second reactor, wherein the temperature in the second reactor is adjusted to 42 to 80°C,
optionally B3) transferring of mixture from the second reactor to a third reactor, wherein the temperature in the third reactor is adjusted to 42 to 80°C,
optionally B4) transferring of mixture from the third reactor to a fourth reactor, wherein the temperature in the fourth reactor is adjusted to 42 to 80°C, and
C) a transfer of mixture, preferably from the last reactor, to a precipitation tank, wherein the precipitation tank contains water or an aqueous solution with up to 55 wt.%, preferably 25 to 45 wt.%, of nitric acid at a temperature between 0 and 25°C, thereby precipitating a precipitate containing 4,6-dinitrobenzofuroxane.

4. Process at least according to claim 3, **characterized in that** the temperature in the first reactor during B) is adjusted, in particular by cooling, to 45 to 85°C, preferably to 50 to 80°C, particularly preferably to 55 to 72°C.

5. Process according to at least one of claims 3 or 4, **characterized in that** the temperature in the second and/or optionally third and/or optionally fourth reactor is adjusted to 50 to 72°C, particularly preferably to 55 to 63°C, particularly by cooling.

6. Process according to at least one of claims 3 to 5, **characterized in that** the average residence time of the mixture or of a molecule of the mixture in each reactor is 3 to 30 minutes, preferably 4 to 10 minutes.

7. Process according to at least one of claims 3 to 6, **characterized in that** the mixture is cooled between the last reactor and C), preferably by at least one further reactor with cooling and/or at least one shell-and-tube exchanger, preferably to a temperature of from 35 to 5°C, preferably from 25 to 10°C.

8. Process according to at least one of claims 1 to 7, **characterized in that** the process comprises precipitation of a precipitate, wherein the precipitate comprises 4,6-dinitrobenzofuroxane, wherein the precipitation is carried out by diluting the concentrated nitric acid with water or aqueous solution with up to 55 wt. %, preferably 25 to 45 % by weight, of nitric acid, and wherein the average grain size or particle size of the 4,6-dinitrobenzofuroxane or the precipitate is adjusted via the temperature of the water or the aqueous solution.

9. Process at least according to claim 8, **characterized in that**
i) by a temperature between 10 and 15°C an average grain size or particle size of 100 to 150 µm or
ii) by a temperature below 10°C an average grain size or particle size of less than 100 µm or
iii) by a temperature of 15 to 25°C, preferably around 20°C, an average grain size or particle size of 150 to 200 µm
is adjusted.

## Revendications

1. Procédé continu de synthèse du 4,6-dinitrobenzofuroxane, comprenant la nitration du benzofuroxane et/ou du 4-nitrobenzofuroxane et/ou du 6-nitrobenzofuroxane dans de l'acide nitrique concentré à une température de 42 à 90 °C, l'acide nitrique concentré contenant de 60 à 100 % en poids d'acide nitrique,
**caractérisé en ce que** l'on n'utilise pas d'acide sulfurique pour la nitration.

2. Procédé selon la revendication 1,
**caractérisé en ce que** l'on n'utilise pas d'anhydride acétique pour la nitration.

3. Procédé selon la revendication 1 ou 2,
le procédé comprenant au moins une des étapes choisies parmi A), B1), B2), B3), B4) et C), de préférence au moins les étapes A), B1), B2) et C), consistant à :
A) initialiser le procédé en mélangeant de l'acide nitrique concentré et du benzofuroxane et/ou du 4-nitrobenzofuroxane et/ou du 6-nitrobenzofuroxane dans un premier réacteur, en formant un mélange, l'acide nitrique concentré contenant de 60 à 100 % en poids d'acide nitrique,
B) poursuivre le procédé
B1) en ajoutant d'avantage d'acide nitrique et de benzofuroxane et/ou de 4-nitrobenzofuroxane et/ou de 6-nitrobenzofuroxane, de préférence dans le premier réacteur et/ou le mélange, pour former un autre mélange, l'acide nitrique concentré contenant de 60 à 100 % en poids d'acide nitrique, et la température dans le premier réacteur étant réglée à une valeur de 42 à 90 °C pendant B),
B2) en transférant le mélange, de préférence du premier réacteur, à un deuxième réacteur, la température dans le deuxième réacteur étant réglée à une valeur de 42 à 80 °C,
éventuellement B3) en transférant le mélange du deuxième réacteur à un troisième réacteur, la température dans le troisième réacteur étant réglée à une valeur de 42 à 80 °C,
éventuellement B4) en transférant le mélange du troisième réacteur à un quatrième réacteur, la température dans le quatrième réacteur étant réglée à une valeur de 42 à 80°C, et
C) transférer le mélange, de préférence du dernier réacteur, à une cuve de précipitation, ladite cuve de précipitation contenant, à une température comprise entre 0 et 25 °C, de l'eau ou une solution aqueuse contenant jusqu'à 55 % en poids, de préférence 25 à 45 % en poids, d'acide nitrique, ce qui permet de précipiter un précipité contenant du 4,6-dinitrobenzofuroxane.

4. Procédé au moins selon la revendication 3,
**caractérisé en ce que** la température dans le premier réacteur est réglée pendant B), en particulier par refroidissement, à une valeur de 45 à 85 °C, de préférence à une valeur de 50 à 80 °C, de manière particulièrement préférée à une valeur de 55 à 72 °C.

5. Procédé au moins selon l'une des revendications 3 ou 4,
**caractérisé en ce que** la température dans le deuxième et/ou éventuellement le troisième et/ou éventuellement le quatrième réacteur est réglée à une valeur de 50 à 72 °C, de manière particulièrement préférée à une valeur de 55 à 63 °C, en particulier par refroidissement.

6. Procédé au moins selon l'une des revendications 3 à 5,
**caractérisé en ce que** le temps de séjour moyen du mélange ou d'une molécule du mélange dans chaque réacteur est de 3 à 30 minutes, de préférence de 4 à 10 minutes.

7. Procédé au moins selon l'une des revendications 3 à 6,
**caractérisé en ce que** le mélange est refroidi entre le dernier réacteur et C), de préférence par au moins un autre réacteur avec refroidissement et/ou par au moins un échangeur à faisceau de tubes, de préférence à une température de 35 à 5 °C, de préférence de 25 à 10 °C.

8. Procédé au moins selon l'une des revendications 1 à 7,
**caractérisé en ce que** le procédé comprend une précipitation d'un précipité, ledit précipité contenant du 4,6-dinitrobenzofuroxane, ladite précipitation étant réalisée par dilution de l'acide nitrique concentré avec de l'eau ou avec une solution aqueuse contenant jusqu'à 55 % en poids, de préférence de 25 à 45 % en poids, d'acide nitrique, et la taille moyenne des grains ou des particules du 4,6-dinitrobenzofuroxane ou du précipité étant réglée par le biais de la température de l'eau ou de la solution aqueuse.

9. Procédé au moins selon la revendication 8,
**caractérisé en ce que**
i) une température comprise entre 10 et 15 °C permet de régler une taille moyenne de grains ou de particules de 100 à 150 µm, et/ou
ii) une température inférieure à 10 °C permet de régler une taille moyenne de grains ou de particules inférieure à 100 µm, et/ou
iii) une température de 15 à 25 °C, de préférence d'environ 20 °C, permet de régler une taille moyenne de grains ou de particules de 150 à 200 µm.
